# EUROPEAN PATENT APPLICATION

(11) **EP 1 167 533 A1**
(43) Date of publication of application: **02.01.2002**
(21) Application number: 00202204.4
(22) Date of filing: 23.06.2000
(51) Int. Cl.: C12N 15/86, C12N 15/861, C12N 5/10, C12N 15/34, C07K 14/075

(54) **Methods and means for the complementation of viral protein expression in stable cell lines**

(71) Applicant: VERENIGING VOOR CHRISTELIJK WETENSCHAPPELIJK ONDERWIJS, NL-1081 HV Amsterdam (NL)
(72) Inventor: van Beusechem, Victor Willem, 1072 HE Amsterdam (NL); Gerritsen, Willem-Ronald, 1422 HE Uithoorn (NL)
(74) Representative: Wittop Koning, Tom Hugo

(57) **Abstract**

Method for the propagation of recombinant virus in packaging cells, comprising the steps of: culturing the packaging cells free of virus introduction of the virus in the packaging cells propagation of the virus within the packaging cells isolating the virus from the packaging cells, wherein at least one gene of the virus, the expression of which leads to a product, being essential for the replication of the virus within the packaging cells is modified to be not functional or at least partly deleted from the said virus, the packaging cells comprise a stably replicating complementing gene, coding for a function complementing for the said essential product, the product being toxic to the said packaging cells, the expression of the said complementing gene being under the control of a transactivator responsive element (TRE), the gene encoding the corresponding transactivator being present in the recombinant virus genome and being expressible in the packaging cells upon infection thereof by the said virus.

## Description

### Field of the invention.

The present invention relates to the propagation of recombinant virus in packaging cells, comprising the steps of:
culturing the packaging cells free of virus
introduction of the virus in the packaging cells
propagation of the virus within the packaging cells
isolating the virus from the packaging cells.
The invention further relates to a DNA construct for cloning into packaging cells, to packaging cells and to a recombinant virus vector.

### Background art

Virus-derived gene transfer vectors, hereinafter referred to as recombinant virus vectors, are generated by displacing parts of the virus genome with heterologous DNA, including but not limited to DNA encoding a therapeutic product, through genetic engineering. It is to be understood that the term recombinant virus vector is also meant to include virus from which parts of the virus genome have been removed without insertion of heterologous DNA. In this specification, the term "recombinant virus" is also meant to encompass recombinant virus vectors.

In general, said parts of the virus genome that are removed or displaced include parts that are essential for at least one step of the virus infectious life cycle. Consequently, the recombinant virus vector is replication deficient, which means that said virus vector alone is incapable of completing the virus infectious life cycle. A special type of recombinant virus vector is the so-called conditionally replicating virus. In conditionally replicating viruses said parts of the virus genome that are removed or displaced include parts that are essential for at least one step of the virus infectious life cycle under certain conditions but not under certain other conditions. Said other conditions could, e.g., mean the conditions that exist in a particular type of cell, in a particular stage of the cell cycle, in a particular developmental stage of the cell, in the presence of particular compounds, and the like. In a particular type of recombinant virus vector said parts of the virus genome that are removed or displaced do not include parts that are essential for at least one step of the virus infectious life cycle. This type of recombinant virus vector, therefore, is a replication competent virus vector. In the case of vectors derived from adenoviruses, said removed or displaced parts usually comprise at least functional parts of the early region 1 (E1).

In order to generate infectious recombinant virus vectors and to propagate said vectors to sufficient amounts for application in, e.g., large-scale protein production or gene therapy, said parts of the virus genome that are removed or displaced need to be complemented *in trans* in the cells in which said virus vector is being generated or produced. Complementing functions are to be understood as factors, such as proteins or peptides, that are provided *in trans* comprising one or more of such proteins/peptides encoded by said parts of the virus genome that are displaced or removed, or functional fragments thereof, where functional means complementing said proteins/peptides/fragments at least in nature but preferably also in amount. It is to be understood, however, that said complementing functions are also meant to include other functional fragments that are not naturally encoded by the said virus genome. Examples of functional fragments that are not naturally encoded by said virus genome but that are explicitly included in the complementing functions according to the present invention are given below. Said functional fragments may e.g. be derived from related viruses; structural capsid proteins from viruses can often be exchanged without loss of capsid integrity between closely related viruses. For example, the fiber proteins of adenoviruses can attach to the capsids of many different other types of adenoviruses, including various human serotypes but also types with different host species. Furthermore, virus surface glycoproteins of enveloped viruses are often very efficiently incorporated in the envelope of various related and unrelated enveloped viruses (so-called phenotypic mixing or pseudotyping). This phenotypic mixing is widespread among a range of enveloped viruses belonging to different families, as reviewed by Zavada (J. Gen. Virol. 63(1982)15-24). Especially the incorporation of the vesicular stomatitis virus G-glycoprotein (VSV-G) in the envelope of recombinant retroviruses or lentiviruses in stead of the endogenous envelope glycoproteins finds use in gene therapy to alter the infection spectrum and stability of said retroviruses or lentiviruses (Burns et al., Proc. Natl. Acad. Sci. U.S.A. 90(1993)8033-8037; Reiser et al., Proc. Natl. Acad. Sci. U.S.A. 93(1996)15266-15271). In addition, parts from distinct virus species can sometimes complement certain virus functions by mechanisms that are not always fully understood. For example, human adenovirus replication is blocked in monkey cells but Simian Virus-40 (SV40) large tumor (T) protein expressed in these monkey cells alleviates the block (Fey et al, J. Virol. 30(1979)201-217). Another example of complementing functions according to the present invention is the natural complementation function of one type of virus for the replication of another type of virus. A type of virus that naturally depends on a complementation function provided by another type of virus is called a dependo-virus. A well-known dependo-virus with utility in gene therapy is the adeno-associated virus (AAV).
Replication of AAV requires functions provided *in trans* by adenovirus or Herpes virus (Berns, In Virology 2nd edition, vol. 2 (1990), p.1743-1763). Finally, it is to be understood that said complementing functions are also meant to include functions provided by hybrid molecules comprising functional fragments of the complementing functions defined above linked to heterologous molecules.

Three methods of complementing parts of the viral genome during recombinant virus vector production have been disclosed, i.e., (1) transfecting or infecting the cells in which said recombinant virus vector is being propagated with an unrelated expression vector encoding said complementing functions, or (2) infecting the cells in which said recombinant virus vector is being propagated with a second related recombinant virus vector, that preferably has been made deficient by genetic engineering in parts of the virus genome other than said complementing functions, or (3) expressing said complementing functions from at least one stable insert in the genome of the cell in which said recombinant virus vector is being propagated. In the latter case, said cell with stable complementing insert is called a packaging cell.

From the perspective of reproducible and safe production of recombinant virus vectors, especially in the context of gene therapy, the use of packaging cells is preferred. This is because the alternative methods have the following disadvantages. The transfection of an unrelated expression vector is difficult to perform with reproducible efficiency, which makes the production process unreliable and expensive. The infection with an unrelated recombinant virus vector can be performed with more reproducible efficiency, but the use of a second recombinant virus vector in the production process is often undesired. The infection with a second related recombinant virus vector has in the prior art often led to the production of recombinant virus vector preparations that are not free from said second related recombinant virus vector. This is because said second related recombinant virus vector will replicate in the infected cells. Disclosed systems in which said second recombinant virus vector is made deficient rely on packaging constraints imposed on said second recombinant virus vector that, however, do not fully block the production of said second recombinant virus vector (e.g., WO96/13597, WO97/32481, and WO98/13510). In addition, the simultaneous presence of two related recombinant virus vectors in the same cell could result in the unwanted occurrence of replication competent virus via homologous recombination between the two related recombinant virus vectors. The presence of a second recombinant virus vector or replication competent virus in recombinant virus vector preparations is often unwanted, in particular if said preparations are to be used in humans.

Packaging cell lines are already available and are widely used for the production of recombinant retrovirus vectors and recombinant adenovirus vectors. Packaging cell lines that complement structural retrovirus proteins include, e.g., PA317 (Miller and Buttimore, Mol. Cell. Biol. 6(1986)2895-2902), □ CRIP (Danos and Mulligan, Proc. Natl. Acad. Sci. U.S.A. 85(1988)6460-6464), GP+envAm12 (Markowitz et al., Virology 167(1988)400-406), PG13 (Miller et al., J. Virol. 65(1991)2220-2224), FLYA13 (Cosset et al., J. Virol. 69(1995)7430-7436), and ProPak (Forestell et al., Gene Ther. 4(1997)600-610) cells, and packaging cell lines that complement adenovirus regulatory proteins encoded by the E1 region include, but are not limited to, 293 (Graham et al., J. Gen. Virol. 36(1977)59-74), 911 (Fallaux et al., Hum. Gene Ther. 7(1996)215-222), and the PER (Fallaux et al., Hum. Gene Ther. 9(1998)1909-1917) cell lines.

To reduce the probability of contamination of recombinant virus vector preparations with replication competent virus, it is furthermore preferred that in packaging cells the insert in the genome that provides the complementing functions does not share functional, if any, sequence homology with the recombinant virus vector, where functional sequence homology means sufficient sequence homology to allow recombination between the homologous parts of said insert and said recombinant virus vector, as disclosed in, e.g., WO97/00326 and WO98/39411, included herein by reference.

The production of recombinant virus vectors using packaging cells usually starts with genetic engineering of at least a part of the virus genome by standard molecular biology techniques known in the art. In some cases, a full-length recombinant virus vector construct comprising all elements required for replication in the packaging cell is made, in other cases the recombinant virus vector genome is separated over two or more constructs that share sequence homology. Next, the virus vector genome (comprised by one or more constructs) is introduced into the packaging cells by DNA transfer methods known in the art, such as calcium phosphate precipitation, DEAE-dextran mediated transfection, lipofectamine mediated transfection, electroporation, and the like. Procedures using a virus vector genome that is separated over more than one construct rely on homologous recombination between the parts of the virus genome that are shared by the constructs to occur in the packaging cells to constitute a complete recombinant virus vector genome.

If the recombinant virus vector integrates into the packaging cell genome and does not exert detrimental effects on its host cell, such as, e.g., is the case for the recombinant retroviruses that are widely employed in gene therapy, stable virus producing cells can be selected and cloned. This results in a pure population of recombinant virus producing cells from which recombinant virus vectors can be harvested at will.

The problem is however, that in many cases the recombinant virus exerts a cytopathic effect on its host cell during the replication process, such as is the case for many recombinant virus vectors that find utility in protein production, gene therapy or vaccination, such as adenovirus vectors, vaccinia virus vectors, and herpes simplex virus vectors. In these situations stable recombinant virus vector producing cells cannot be made. Production of such recombinant virus vectors on packaging cells requires re-infection of new target packaging cells for each cycle of vector expansion. The present invention is of particular importance for the latter type of recombinant virus vector production.

Usually, replication of the recombinant virus vector starts in only a minority of the packaging cells that were subjected to transfer of the DNA encoding said recombinant virus vector. This depends, among other factors, on the efficiency of DNA transfer, the efficiency of the homologous recombination leading to the constitution of a complete recombinant virus vector genome, and the effectiveness of the complementation provided by the packaging cells. Starting from the few packaging cells in which recombinant virus vector replication initially takes place, the recombinant virus vector can subsequently spread to other cells in the culture. The recombinant virus vector can also be isolated from the culture medium of the packaging cells. In some cases, it is preferred to isolate the recombinant virus vector from lysates of the packaging cells in which said recombinant virus vector replicated. The isolated recombinant virus vector can then be used to re-infect new packaging cells to further propagate and expand said recombinant virus vector.

It is generally preferred that the recombinant virus vector is deleted of as much as possible of the viral genome, in particular of as much as possible of the parts of the genome that encode viral proteins. This maximal deletion of the viral genome has two important advantages, i.e., (1) it creates maximal space in the recombinant virus vector for insertion of heterologous DNA for gene transfer, and (2) it minimizes the transfer of viral sequences into the target cell. The latter is particularly important in situations where said viral sequences are expressing viral products in the target cell, especially in the context of gene therapy. Said expression of viral products could be directly toxic to the target cell or elicit an immune response against the target cell, thereby diminishing the duration of the expression of the heterologous DNA, thus limiting the effect of the therapy. The latter is a well-recognized problem in gene therapy using recombinant adenovirus vectors. Hence, there is much interest in the development of adenovirus vector systems using vectors that comprise minimal parts of the viral genome (so-called minimal vectors), as disclosed in, e.g., WO96/13597, WO97/32481, WO98/13510, and WO98/17783. Consequently, there is need in the field for systems that complement the deletions in these minimal vectors. As discussed above, it is preferred that this complementation is provided by packaging cells.

For various applications of recombinant virus vectors, especially for gene therapy, it is furthermore preferred that the natural infection spectrum (tropism) of the virus is altered. A viral attachment protein may be the (although less preferred) wild type protein for the virus, or a modified protein, or be derived from another source, such as another related virus, in order to obtain altered binding specificity. This means that the binding specificity of the recombinant virus can be altered by expressing a suitable, optionally heterologous, attachment protein in the packaging cell. Said attachment protein will be incorporated in the coat of the recombinant virus, resulting in binding specificity of said virus conferred by said attachment protein. Preferably, one or more of the natural genes encoding for an attachment protein are deleted from the virus or made dysfunctional, so that the virus looses its natural specificity.

One approach to accomplish this is by changing the binding specificity of the viral attachment protein through genetic engineering. In this respect, it is of particular interest to develop recombinant virus vectors with modified attachment proteins on their surface that exhibit limited binding only to particular cell types, such as, e.g., cancerous cells or hemopoietic cells (so-called targeted vectors). As a consequence of their specificity, many of said targeted vectors will not bind, and will thus not infect, the currently available packaging cells which will often not express the specific receptor for said targeted vector. Recently, a first generation of targeted adenovirus vectors that do not anymore bind to the wild-type adenovirus receptor was disclosed (Roelvink et al., Science 286 (1999)1568-1571). These vectors carry a peptide tag inserted into the attachment protein which allows the vector to be propagated by employing a packaging cell line that was made to express an artificial receptor for said peptide tag. If, however, targeted vectors are developed with a binding specificity for which packaging cells can not easily be made, an attachment protein capable of binding to the packaging cells need to be provided during vector propagation. Therefore, there is also interest in the field for packaging cells that complement the wild-type or modified viral attachment protein during the generation and propagation of targeted vectors.

As will be clear from the above, there is need for packaging cells that functionally complement many different parts of a virus genome that are necessary for completion of the virus life cycle during recombinant virus vector production, where functional means at least in nature but preferably also in amount. The latter is because limited amounts of complementing protein production in the packaging cells could result in slower vector production and/or production of lower amounts of vector. A major limitation for the generation of such packaging cells is that many proteins, e.g. virus proteins, are toxic to cells. For the purpose of the invention, a protein is regarded toxic to packaging cells when said cells do not allow stable expression of said protein at a level that is needed to functionally complement a missing function in a virus genome. In addition, expression of certain proteins during particular stages of the virus life cycle may interfere with the virus replication and therefore regarded to be toxic for the recombinant virus that is to be produced by the packaging cells. Thus, although the cell may allow high levels of expression of such a protein, said expression during said particular stage of the virus life cycle will not functionally complement for a function that is missing from the genome of said virus. Therefore, both said proteins or protein products that are toxic to the packaging cells and said proteins that disturb certain stages of the virus life cycle within the packaging cells will, for the purpose of the invention, be further referred to as toxic proteins or toxic protein products. A "protein product" is to be understood as a protein that may have been subjected to further processing in order to maturate to a functional product. Such a product can e.g. be a glycosilated protein. Examples of toxic virus proteins include e.g. the adenovirus E4-34k (Halbert et al., J. Virol. 56(1985)250-257), DNA-binding protein (DBP; Klessig et al., Mol. Cell. Biol. 4(1984)1354-1362 ), pTP (depending on the host cell type; Schaack et al., J. Virol. 69(1995)4079-4085), and fiber (Levine and Ginsberg, J. Virol. 1(1967)747-757) proteins, AAV *rep* proteins (Mendelson et al., Virology 166(1988)612-615; Khleif et al., Virology 181(1991)738-741), Human Immunodeficiency Virus (HIV) protease (Kaplan et al, Biomed. Biochem. Acta 50(1991)647-653) and Vpr (Rogel et al, J. Virol. 69(1995)882-888) proteins, herpes simplex virus (HSV) UL41-58K phosphoprotein (Smibert et al., J. Gen. Virol. 73(1992)467-470) and VSV-G (Yang et al., Hum. Gene Ther. 6(1995)1203-1213). During wild-type virus infection of target cells, many toxic virus proteins are therefore only temporarily expressed, thus precluding their interference with essential processes during other stages of the virus replication, and allowing the target cell to survive long enough for the virus to complete its life cycle. In many but not all cases, this temporal expression is an expression during the late stages of the virus life cycle. In summary: expression of toxic virus proteins in stable packaging cell lines may lead to premature death of the cells, precluding the generation of stable packaging cell lines that also complement said proteins, or may interfere with early stages of the virus life cycle, resulting in sub-optimal recombinant virus vector production.

Adenovirus E1-complementing packaging cells that also stably express the toxic adenovirus fiber protein (AdE1/fiber-complementing packaging cells) have already been disclosed (Von Seggern et al., J. Gen. Virol. 79(1998)1461-1468; Legrand et al., J. Virol. 73(1999)907-919) and WO98/13499, included by reference herein. These stable AdEl/fiber-complementing packaging cells express fiber protein at levels that are much lower than the fiber protein expression levels found in AdE1-complementing packaging cells that contain a replicating fiber-positive adenovirus vector. As a direct result, the propagation of a fiber-negative adenovirus vector on said AdE1/fiber-complementing packaging cells was significantly delayed. Viral plaques were observed 15-18 days after infection, where fiber-positive adenovirus vectors produced plaques on E1-complementing packaging cells within 5-7 days (Von Seggern et al., J. Virol. 73(1999)1601-1608). In addition, the titers of infectious recombinant fiber-negative adenovirus vectors that could be produced on said AdE1/fiber-complementing packaging cells were significantly reduced as compared to the infectious titers of fiber-positive adenovirus vectors produced on AdE1- or AdE1/fiber-complementing packaging cells (30-fold, Von Seggern et al., J. Virol. 73(1999)1601-1608; 172-fold, Legrand et al., J. Virol. 73(1999)907-919). Moreover, the initial production of the recombinant E1/fiber-deleted adenovirus vector on AdE1/fiber-complementing packaging cells by co-transfecting said cells with overlapping plasmid constructs was very inefficient. Only one culture dish out of 58 yielded the infectious vector, while a control co-transfection using a fiber-positive plasmid construct gave infectious vector in 9 out of 9 dishes (Von Seggern et al., J. Virol. 73(1999)1601-1608).

AdE1-complementing packaging cells that in addition stably express the AdE4 proteins (AdE1/E4-complementing packaging cells) were also disclosed (Lusky et al., J. Virol. 72(1998)2022-2032). Said AdE1/E4-complementing packaging cells express the AdE4 proteins from their endogenous AdE1-dependent promoter. Because said packaging cells stably express the AdE1 proteins that trans-activate the AdE4 promoter, the AdE4 proteins are also stably expressed. The infectious titers of E1/E4-deleted adenovirus vectors on said packaging cells were, however, 1,000 to 10,000-fold lower than the titers of E1-deleted adenovirus vectors. This may be explained by the fact that at least one of the AdE4 proteins, the E4ORF6-encoded E4-34k protein, is a toxic protein.

Thus, it is to be clearly understood from the above that although it is sometimes possible to stably express toxic viral gene products in packaging cells at low levels, this expression is often insufficient for reproducible and efficient recombinant viral vector production. Most probably, during expansion of said packaging cells only a subpopulation of low toxic protein-expressing cells survives. This was shown to be the case for adenovirus fiber protein expressed in AdE1-complementing packaging cells herein (see example 2). Therefore, there is a need for packaging cell systems that allow high-level expression of the toxic viral protein during the correct stage of recombinant vector propagation, but low-level expression of said protein during generation and expansion of the packaging cells. This can be accomplished, e.g., by directing the expression of said toxic protein by a regulatable (also referred to as "inducible", "repressible", or "suppressible") promoter. A regulatable promoter is described as a promoter wherein the rate of RNA polymerase binding and RNA transcription can be modulated by an external signal, where the term external means having its origin outside of the expression cassette of said toxic protein. Examples of said external signal include, but are not limited to, the addition or deprivation of a chemical compound, a shift in temperature, a decreased oxygen concentration, and the like. A special kind of regulatable promoter is a tissue- or cell type-specific promoter, where said external signal is provided by a protein that is only present in a particular type of cell or tissue. Another special kind of regulatable promoter is a so-called Transactivator Response Element (TRE). Said TRE is a first component of a transactivation system that comprises as a second component a transactivator protein. Said transactivator protein is capable of binding with specificity to said TRE, thereby regulating the transcription of a gene linked to said TRE. Said transactivation system may optionally comprise as a third component a compound that influences the binding of said transactivator protein to said TRE, thereby further regulating said transcription.

Many different systems to control gene expression in mammalian cells have been described in the art, some non-limiting examples of which are mentioned below and included herein by reference. Limited success was originally obtained using expression systems that rely on inducible eukaryotic promoters responsive to, e.g., metal ions or hormones (Hynes et al., Proc. Natl. Acad. Sci. U.S.A. 78(1981)2038-2042; Brinster et al., Nature 296(1982)39-42; Searle et al., Mol. Cell. Biol. 5(1985)1480-1489; Israel and Kaufman, Nucleic Acids Res. 17(1989)2589-2604) and with inducible bacterial repressor systems that were modified to function in mammalian cells (Hu and Davidson, Cell 48(1987)555-566; Brown et al, Cell 49(1987)603-612; Deuschle et al., Proc. Natl. Acad. Sci. U.S.A. 86(1989)5400-5404). Later, systems with greater specificity were designed that employ chimeric transactivator proteins consisting of a DNA-binding domain of a bacterial or yeast protein fused to a transcription activator domain of a mammalian protein or of a protein from a virus that replicates in mammalian cells (chimeric transactivation systems). Said chimeric transactivation systems have greater specificity because the chimeric transactivator protein recognizes a target DNA sequence that is of bacterial or yeast origin and, thus, is not naturally present in the mammalian genome. Below a number of examples of said chimeric transactivation systems is given. A first family of chimeric transactivator proteins is the *lacI* activator protein (LAP) family. LAPs comprise parts of the bacterial *lac* repressor protein linked to the transactivator domain from the herpes simplex virus virion protein-16 (VP16). They activate the transcription from an operon containing spaced sets of *lac* operators located upstream or downstream from a minimal promoter. The transcription is regulated by the compound isopropyl-□ -D-thiogalactopyranoside (IPTG). IPTG binds to LAP, thereby interfering with its multimerization into an active form. LAP-dependent expression of reporter genes was regulated over a range of more than 1,000 fold in stable cell lines (Baim et al., Proc. Natl. Acad. Sci. U.S.A. 88(1991)5072-5076). Another example of a chimeric transactivation system utilizes a fusion protein of the transactivator domain from VP16 with the DNA-binding domain of LexA, and LexA binding sites introduced into the promoter (Brent et al., Cell 43(1985)729-736). A chimeric transactivator system that has found wider use is based on a fusion between the transactivator domain of VP16 with part of the bacterial tet repressor protein tetR (tTA; Gossen and Bujard, Proc. Natl. Acad. Sci. U.S.A. 89(1992)5547-5551) or with part of a tetR derivative that has reverse DNA binding properties as compared to tetR (rtTA; Gossen et al., Science 268(1995)1766-1769). These chimeric transactivator proteins stimulate minimal promoters fused to tet operator (*tet*O) sequences. Transcription regulation is mediated by tetracycline (Tc) or one of its derivatives, such as doxycycline or anhydrotetracycline. Binding of Tc or one of its derivatives to tTA or rtTA induces a conformational change in the protein. The conformational change of tTA prevents its binding to *tet*O and thereby transactivation, whereas, on the other hand, the conformational change of rtTA is required for binding and transactivation. These tetracycline-responsive transactivation systems, that are generally referred to as *tet*O/tTA and *tet*O/rtTA, respectively, were reported to regulate gene expression in mammalian cells over three to five orders of magnitude.

A chimeric transactivation system that can be regulated *in vivo* in response to an orally administered drug uses one of the chimeric regulator proteins GLVP or GLp65. GLVP and GLp65 both contain the ligand binding domain of a mutant human progesterone receptor (hPRB891), that has lost its ability to bind progesterone or other endogenous hormones but can still bind the progesterone antagonist RU486, and the DNA-binding domain, nuclear localization signal and dimerization function of the yeast transactivator protein GAL4. In GLVP these domains are fused to VP16 (Wang et al., Proc. Natl. Acad. Sci. U.S.A. 91(1994)8180-8184), while GLp65 contains the activation domain of human p65, a member of the NF□ B family of proteins (Burcin et al., Proc. Natl. Acad. Sci. U.S.A. 96(1999)355-360). Only in the presence of RU486 (also known as mifepristone), GLVP or GLp65 binds to GAL4-binding sites introduced into a promoter, thereby transactivating the expression of a linked gene. The reported range of expression regulation for this type of transactivation system is approximately 50-fold. Another chimeric transactivator system that can be regulated in vivo in response to an orally administered drug uses so-called dimerizer drugs, such as synthetic dimers of FK506 or rapamycin (Spencer et al, Science 262(1993)1019-1024; Ho et al, Nature 382(1996)822-826; Rivera et al., Nature Med., 2(1996)1028-1032). Said dimerizer drugs function as a heterodimerizing ligand for, on one side, a fusion protein of a DNA-binding domain and a dimerizer drug-binding domain and, on the other side, a fusion protein of a transactivator domain and a dimerizer drug-binding domain. The interaction with the dimerizer drug results in the juxtapositioning of the DNA-binding and transactivator domains, thereby creating a functional chimeric transactivator protein.

Regulatable gene expression can also be obtained in mammalian cells by employing a transactivation system consisting of a promoter from a distant species and a counterpart RNA polymerase enzyme that specifically binds to said promoter, where said mammalian cells do not naturally express a counterpart RNA polymerase for said promoter. A non-limiting example of this type of transactivation system consists of the RNA polymerase enzyme from bacteriophage T7 and a counterpart promoter comprising the specific bacteriophage T7 polymerase DNA binding sequence 5'-TAATACGACTCACTATAGG-3' (e.g., Studier et al., Methods in Enzymology 185(1990)60-89; Moss et al., Nature 348(1990)91-92). The bacteriophage T7 polymerase transactivation system has already been shown to function in the context of recombinant adenovirus vectors (Tomanin et al., Gene 193(1997)129-140).

Apart from systems to control gene expression in mammalian cells that rely on regulatable promoters as described above, activation or inactivation of expression can also be achieved by using site-specific recombinases (reviewed by Kilby et al., Trends Genet. 9(1993)413-421), including but not limited to Cre recombinase derived from bacteriophage P1 (Sternberg and Hamilton, J. Mol. Biol. 150(1981)467-486) and FLP recombinase (Broach and Hichs, Cell 21(1980)501-508). In contrast to systems using regulatable promoters that may provide a quantitative regulation of expression dependent on the amount of a drug, gene expression under the control of a recombinase only allows for an efficient switch between an off and an on-state. Although the on/off-switch is in principle not irreversible, recombinase systems are usually only designed for efficient one-way activation or inactivation of gene expression. Below the Cre*-loxP* system is explained as a non-limiting example of a way to use site-specific recombinases to regulate gene expression. The Cre-*loxP* system requires only two components, i.e., the 38 kDa Cre recombinase protein and the 34 bp *loxP* target DNA sequence. Said 34 bp *loxP* target DNA sequence comprises an 8 bp asymmetric core sequence flanked by two 13 bp inverted repeats. Cre protein binds to the 13 bp inverted repeats of two *loxP* sites and catalyzes the precise recombination between the 8 bp asymmetric core sequences of said two *loxP* sites. If a target DNA molecule carries two parallel *loxP* sites, as defined by their core sequences, the recombination between said parallel *loxP* sites results in the excision of the DNA sequences intervening said parallel *loxP* sites. One complete *loxP* site remains in the recombined target DNA molecule and the intervening sequences are released as a circular molecule with one *loxP* site. Cre recombinase also catalyzes the reverse reaction, where sequences carried on a circular DNA molecule with one *loxP* site are inserted into a different DNA molecule with one *loxP* site. Activation of gene expression can be brought about by Cre-mediated excision of a transcription termination cassette with flanking *loxP* sites from the region between the promoter and the open reading frame of the gene. Said transcription termination cassette usually comprises a polyadenylation signal, preventing the formation of full-length mRNA containing the open reading frame of the gene. For the purpose of the present invention, a site-specific recombinase is also regarded as a transactivator protein and a region comprising a promoter and a transcription termination cassette with flanking target sites for said site-specific recombinase is also included in the term TRE. Hence, together they comprise a transactivation system. Said activation of gene expression mediated by the action of a site-specific recombinase protein is called Recombination-Activated Gene Expression (RAGE) and will be further referred to as such hereinafter. Cre-mediated excision can also be used to inactivate gene expression, when the *loxP* sites flank an expression cassette or at least an essential DNA fragment within said expression cassette. The Cre-*loxP* system was successfully used in cultured mammalian cells and in transgenic mice *in vivo* (e.g., Sauer and Henderson, Nucleic Acids Res. 17(1989)147-161; Lakso et al., Proc. Natl. Acad. Sci. U.S.A. 89(1992)6232-6236; Gu et al., Science 265(1994)103-106). In addition, Cre expressed from a recombinant virus vector could mediate RAGE of genes inserted in the genome of cultured mammalian cells that were infected with said recombinant virus vector (Kanegae et al., Nucleic Acids Res. 19(1995)3816-3821). RAGE was also achieved following co-infection of cultured mammalian cells with a Cre-expressing recombinant virus vector and a second virus vector carrying the inducible target gene with *loxP*-flanked transcription termination cassette (Anton and Graham, J. Virol. 69(1995)4600-4606). The Cre-*loxP* system was furthermore shown to function after *in vivo* infection of transgenic mice carrying an inducible target gene with *loxP*-flanked transcription termination cassette in their genome with a Cre-expressing recombinant virus vector (Wang et al., Proc. Natl. Acad. Sci. U.S.A. 93(1996)3932-3936).

The use of transactivation systems for the regulation of expression of toxic proteins in the context of recombinant virus vectors has until now primarily been confined to transient systems. Yoshida et al (Biochem. Biophys. Res. Com. 232(1997)379-382) transiently expressed the toxic VSV-G protein and the retrovirus *gag* and *pol* proteins in cells after co-infection of said cells with replication-deficient recombinant adenovirus vectors carrying *tet*O-dependent expression cassettes for said proteins and a recombinant adenovirus vector expressing a variant of tTA protein with a nuclear localization signal. This resulted in the production of VSV-G pseudotyped retroviruses by said cells. Hu et al (Cancer Res. 57(1997)3339-3343) constructed a single recombinant AdE1-adenovirus vector that expresses the tTA protein and carries a *tet*O-dependent expression cassette for tumor necrosis factor □ (TNF-□ ). Expression of the TNF-□ protein - which is detrimental for the viability of the transformed cells - was effectively suppressed in the presence of Tc, thus allowing the production of the recombinant adenovirus vector in transformed packaging cells. Massie et al (J. Virol. 72(1998)2289-2296) used a similar strategy to express another toxic protein (a deletion mutant of HSV ribonucleotide reductase). The *tet*O-dependent expression of this protein from a recombinant adenovirus vector was regulated using Tc or one of its derivatives in cells that were either carrying a stable tTA expression insert in their genome or that were co-infected with a tTA-expressing recombinant adenovirus vector. The Cre-*loxP* system has been applied to generate recombinant adenovirus vectors that express one of the apoptosis inducing proteins Bcl-2 or Fas-ligand (Sato et al, Mol. Cell. Neurosci. 12(1998)65-78; Okuyama et al., Gene Ther. 5(1998)1047-1053). A Cre-expressing adenovirus vector and a second adenovirus vector carrying the gene for one of said apoptosis inducing proteins with a *loxP*-flanked transcription termination cassette were propagated separately. Bcl-2 or Fas-ligand expression was activated transiently in cells co-infected with both recombinant adenovirus vectors *in vitro* or *in vivo.* Thus, transactivation expression systems have in the prior art been used for the production of recombinant virus vectors that are capable of introducing and expressing a toxic product in target cells. The expression was made regulatable to prevent expression of said toxic product during production of said recombinant virus vector in packaging cells.

In contrast, for the complementation of toxic virus proteins during recombinant virus vector production the expression of said toxic virus proteins is especially required in the packaging cells. Several stable packaging cell lines that employ regulatable promoters to express viral proteins during recombinant virus production have been disclosed. Brough et al. (Virology 190(1992)624-634) made adenovirus DBP-complementing cells by utilizing the Mouse Mammary Tumor Virus (MMTV) LTR-promoter that can be induced by addition of dexamethasone. The same group (J. Virol. 70(1996)6497-6501) as well as Gao et al (J. Virol. 70(1996)8934-8943) made AdE1/E4ORF6-complementing cells in which the AdE4ORF6 expression is driven either by the metallothionein promoter that is responsive to divalent cations or by the MMTV LTR-promoter. Lusky et al. (J. Virol. 72(1998)2022-2032) generated AdE1/E2A-complementing cells in which the AdE2A expression is driven by the MMTV LTR-promoter, and AdE1/E4ORF6+7-complementing cells in which the expression of the AdE4ORF6+7 proteins is regulated using the *tet*O/tTA expression system, where transcription is suppressed in the presence of tetracycline or doxycycline. The latter method was also used by He et al (Proc. Natl. Acad. Sci. U.S.A. 95(1998)2509-2514) to regulate the expression of the complete AdE4 region in AdE1-complementing packaging cells. Yang et al (J. Virol. 68(1994)4847-4856) employed the metallothionein promoter to inducibly express the AAV *rep* proteins in packaging cells and Hoelscher et al (J. Virol. 68(1994)7169-7177) regulated the expression of the AAV *rep* proteins in packaging cells using the MMTV LTR-promoter. Recently, Kafri et al. (J. Virol. 73(1999)576-584) disclosed a packaging cell line for lentivirus vectors in which the expression of most HIV genes, some of which are toxic, and of the toxic VSV-G are regulated using the *tet*O/tTA expression system.

The Cre-*loxP* system was used by Arai et al. (J. Virol. 72(1998)1115-1121) to transactivate the expression of VSV-G in retrovirus packaging cells. RAGE was accomplished by infecting the packaging cells during recombinant retrovirus production with a Cre-expressing adenovirus vector. This inducible packaging cell system is importantly different from those discussed above in that the production of the recombinant virus vector (a retrovirus vector) requires infection of the packaging cell with a second, unrelated recombinant virus vector (an adenovirus vector). From the perspective of recombinant virus vector production for use in gene therapy or vaccination the introduction of a second, unrelated, virus vector during the production process is undesirable.

An important disadvantage of the use of the regulatable expression systems for toxic virus proteins in packaging cells disclosed so far is that in all cases the switch from no or low toxic virus protein expression to high toxic virus protein expression needs to be brought about by an action of the person performing the recombinant virus vector production. A compound or composition is added to or removed from the culture medium, the cultures are placed at a different temperature or infected with a second virus vector, or the like. Said action can only be performed effectively if (1) the correct timing for the expression induction is known, (2) the lag-time between said action and said switch is reproducible, and (3) all cells in the culture are synchronized with respect to the recombinant vector replication in said cells. Although it can be envisaged that those skilled in the art may fulfil the first two requirements by proper investigation, a production process comprising infection of the packaging cell with the recombinant vector followed or preceded by induction of the toxic virus protein expression within a tight time-schedule will be more complicated than a standard recombinant virus vector production method known in the art. Moreover, the third requirement will certainly not be met during all stages of the recombinant virus vector production process if stable recombinant virus vector producing cells cannot be made. In particular, as discussed above, in the early phase of the recombinant virus vector production process only very few cells will contain replicating virus vectors, while most cells in the culture need to remain viable to serve as target cells for viruses that start to spread in the culture later. In this respect, it is of note that it sometimes takes several weeks before recombinant adenoviruses form plaques after co-transfection of two constructs that together comprise the recombinant adenovirus genome and require homologous recombination to constitute a replicating genome (Von Seggern et al., J. Virol. 73(1999)1601-1608). Therefore, in the few cells containing replicating vectors the toxic virus protein expression needs to be induced, while in all other cells in the culture said expression needs to remain suppressed, until also these cells are infected by the spreading recombinant virus vector. None of the regulatable complementation systems in packaging cells disclosed so far allows induction of the complementing toxic virus protein only in those cells of the culture in which recombinant virus vector replication is ongoing, while all other cells remain unaffected. The present invention provides a regulatable complementation system that fulfils the need to induce toxic virus protein expression only in cells in which the recombinant virus vector is replicating.

### Brief description of the invention.

The invention provides an improved method for the propagation of recombinant virus, such as recombinant virus vector, in packaging cells, wherein the above-mentioned drawbacks are avoided. and is characterized in that at least one gene of the virus, the expression of which leads to a product, being essential for the replication of the virus within the packaging cells is modified to be not functional or at least partly deleted from the said virus,
the packaging cells comprise a stably replicating complementing gene, coding for a toxic complementing protein product functionally complementing for the said essential product, the expression of the said complementing gene being under the control of a transactivator responsive element (TRE),
the gene encoding the corresponding transactivator being present in the recombinant virus genome and being expressible in the packaging cells upon infection thereof by the said virus.

"Functionally complementing" is to be understood as the capability of the toxic protein product to substitute for the product, essential for virus replication, such that the virus is capable to use the complementing product for successful replication.

In a preferred embodiment, the gene encoding the product, essential for virus replication is deleted from the viral genome. Preferably said product, optionally modified, is toxic and encoded by the genome of the packaging cells under the control of a TRE. In that case, the complementing function is directly derived from the viral genome. As is explained earlier, the term "toxic" relates to the complementing function, and not necessarily to the viral product, necessary for the replication thereof in packaging cells. However, said viral product may be toxic, in particular for the packaging cell, and be expressed by the said packaging cell under the control of a TRE.

In the method according to the invention, the viral protein, essential for virus replication, is not encoded by the virus genome, however, a function complementing for the said protein, essential for virus replication, being toxic to the packaging cells, is encoded by the genome of the packaging cells, operably linked to a transactivator responsive element (TRE). The virus genome, however, comprises a transactivator that is capable to interact with the said TRE, so that the expression of the gene encoding the toxic protein is only induced upon the presence of the virus in the packaging cells, during at least a part of the replication of the virus, e.g. the late phase in case of a late adenovirus derived complementing function.

"Stably replicating" means that during cell division, the genetic material is transferred to both daughter cells. Preferably, the genetic material is incorporated in the genome of the cell, but the skilled person will understand that stable replication is also possible when the respective gene is e.g. present on a plasmid, for the said expression, viral components, present in the said packaging cells may be essential.

An "expressible" gene means that said gene can be expressed, in this case in and by the machinery of the packaging cells; for the said expression, viral components, present in the said packaging cells may be essential. The said gene does however not have to be expressed immediately upon infection, but may be subjected to additional regulatory control, e.g. intrinsic to the virus or to the host. It is also possible that the said gene is expressed only in the presence of a certain compound in the medium, such as an antibiotic.

Preferably, the packaging cell is itself incapable of producing a transactivator that functionally binds the TRE, although certain binding that may induce the expression of the gene to subtoxic levels of the toxic protein may be allowed. "Functionally binding" is to be understood as binding of the transactivator to the TRE leading to expression, or to improved expression compared to the level of expression in absence of the transactivator, of the gene operably linked to the TRE.

"Operably linked" means that the gene encoding the toxic protein is coupled to the TRE in such a manner that the expression of the said gene is induced upon binding of the proper transactivator to the TRE.

Preferably, the recombinant virus comprises heterologous nucleotide sequences, said heterologous sequences being chosen from the group consisting of sequences comprising the transactivator gene, sequences encoding a therapeutic product, or any combination thereof. When the virus comprises a gene for a therapeutic product, the virus can be used for gene transfer of the said gene. "Therapeutic product" is to be understood as a protein or peptide having a curing effect, or as a starting material for the preparation of a therapeutic drug, by isolating the product and bring it in an administratable form. It is advantageous when the transactivator gene is heterologous in order to avoid transactivating the expression of undesired genes in the host cells.

It is an important objective of the present invention to provide methods and means to express certain proteins in packaging cells during recombinant virus vector replication in said packaging cells, but not or at least in much lower amounts in said packaging cells prior to recombinant virus vector replication. It is to be understood that the term replication is meant to include all steps of the virus life cycle that occur in the host cell, starting with infection of the cell and ending with release of newly produced virus from the cell. For double-stranded DNA viruses, the replication can be divided sequentially in infection, an early phase, a DNA-replication phase, a late phase, and release. Viral proteins that are expressed uniquely or primarily in the early or late phase of replication are referred to as early and late viral proteins, respectively. The invention utilizes a transactivation system, where a replicating recombinant virus vector provides the transactivator protein and a complementing protein is expressed from a stable insert in the packaging cell that comprises the corresponding TRE that is responsive to said activator protein provided by said recombinant virus vector.

Hereinafter, in several embodiments of the invention a number of ways to provide said methods and means are given, exemplified for the preferred recombinant virus vectors, but skilled artisans will be able to transfer these teachings to other virus vectors, packaging cells, transactivation systems, and methods to produce these vectors or cells without departing from the present invention. It is also to be clearly understood that any product prepared according to the invention, as well as any use of said product, is part of the present invention.

The definitions of the terms used in the invention specification and claims are themselves clearly understood in the art.

In one embodiment of the present invention, a recombinant DNA construct is provided that comprises an open reading frame (ORF) for a toxic protein functionally linked to a TRE, where it is preferred that said toxic protein expressed by said recombinant DNA construct is biologically active. Said TRE comprises one or more binding sites for a transactivator protein, where it is preferred that said binding sites do not naturally occur in mammalian cells, in particular in human cells and in the cells used for the production of recombinant virus vectors according to the invention. In a variation of the invention, the recombinant DNA construct carries more than one ORF, each encoding a different toxic protein, functionally linked to the same TRE or to different TREs. Non-limiting examples of TREs that are useful in the invention are promoters that include binding sites for the bacterial *lac* repressor protein, the bacterial LexA protein, the bacterial tet repressor protein, the yeast transactivator protein GAL4, or certain RNA polymerases such as bacteriophage T7 RNA polymerase, and TREs that comprise binding sites for site-specific recombinases such as Cre recombinase and the like.

According to the invention, said toxic protein is preferably a viral protein, but may also be a hybrid protein that comprises a functional fragment thereof. Said viral protein may be a regulatory protein or a structural protein. In one variation, said viral protein is a protein of which the expression is regulated during normal virus replication in the host cell, where it is preferred that said protein is only expressed during a part of said normal virus replication. In one aspect of the invention, said expression is induced after virus DNA replication in the host cell. In a preferred embodiment, said viral protein is a DNA virus protein or comprises a functional fragment thereof. "Functional fragment" is to be understood as a fragment of the protein that still is capable of exerting the required function of the complete protein.

It is particularly preferred that said viral protein is a protein from a virus selected from the group consisting of adenovirus, herpes simplex virus, vaccinia virus, adeno associated virus, Epstein-Barr Virus, SV-40, cytomegalovirus, and Human Papilloma Virus. In a further embodiment, said viral protein is an adenovirus protein of which the expression is mainly confined to the late phase of adenovirus replication. Examples of adenovirus proteins are proteins selected from the group consisting of hexon, fiber, penton base, 100K protein, core polypeptides V, VI, VII, and VIII, 23K-protease, and the like.

The present invention also provides cells that comprise a stable insert in their genome of the recombinant DNA construct according to the invention, where it is preferred that said cells are immortalized and where it is further preferred that said cells constitute a clonal population, which will be further referred to as a cell line. In order to avoid expression of the complementing gene(s), said cells preferably do not stably produce a functional transactivator protein for said recombinant DNA construct. In one variation of the invention, said cells carry a single insert of said recombinant DNA construct in their genome, in another variation they carry multiple insertions of said recombinant DNA construct. Said multiple insertions may each represent the same recombinant DNA construct, or may alternatively comprise different recombinant DNA constructs according to the invention each carrying an ORF for a different toxic protein.

The cells of the invention support replication of a recombinant virus vector according to the invention. The cells of the invention may be derived from prokaryotic cells, but it is preferred that they are derived from eukaryotic cells. In several contemplated embodiments, the use of mammalian cells, and more particularly of human cells, is preferred. In preferred embodiments, the cells of the invention are derived from virus packaging cells. Non-limiting examples of cell lines that are useful in various embodiments of the invention to serve as parental cells to derive the cells of the invention are HeLa, A549, OVCAR-3, BHK, CHO, Vero, CV-1, COS-1, COS-7, human embryo kidney (HEK) cells, human embryo retina (HER) cells, 293, 911, and the PER cell lines.

In a further embodiment, the invention provides cells that in addition to the recombinant DNA construct according to the invention carry one or more complementing constructs in their genome, such as to provide complementation for more than one function deleted from the recombinant virus vector. In this way, even more of the original genome of the virus can be deleted leading to an optimal minimal vector, providing more place for insertion of one or more exogenous sequences for transfection. In one variation, the protein encoded by such an additional complementing construct is a regulatory protein for virus replication, where it is preferred that said regulatory protein does not functionally bind to the TRE of the recombinant DNA construct according to the invention. In another variation said protein is a structural virus protein or comprises a functional part thereof. In one aspect of the invention, the protein encoded by said second complementing construct is a toxic protein. The expression of the protein encoded by said second complementing construct is driven by a stable promoter or by a regulatable promoter. In a particular embodiment of the invention, the expression of the protein encoded by said second complementing construct is regulated according to the present invention.

The present invention also discloses adenovirus packaging cells that carry a recombinant DNA construct according to the invention in their genome. In one variation, the adenovirus packaging cells according to the invention, in addition to the protein encoded by the DNA construct according to the invention, also complement the polypeptides of the E1-region, where it is preferred that said packaging cells are derived from 293, 911, or PER cells. In another variation, said adenovirus packaging cells further complement other adenovirus regulatory proteins in addition to those encoded by the E1-region, such as the 100K protein and the proteins encoded by regions selected from the group consisting of E2A, E2B, and E4. In yet another variation, said adenovirus packaging cells in addition to the proteins encoded by the E1-region further complement adenovirus structural proteins, such as the proteins selected from the group consisting of hexon, penton base, fiber, core polypeptides V, VI, VII, and VIII, and 23K-protease.

The invention also provides adenovirus E1-complementing packaging cells that carry a recombinant DNA construct according to the invention in their genome, where said recombinant DNA construct comprises an ORF for an adenovirus fiber protein. In one variation, the nucleotide sequence in said ORF is altered such that the encoded fiber protein is targeted towards a specific receptor, where it is preferred that this modification alters the binding specificity of the fiber for different cell types. Incorporation of such a modified fiber in the virus capsid provides the virus with a new or altered tropism.

In another embodiment, the present invention provides a recombinant virus vector, of which at least one gene coding for a product being essential for the replication of the virus in cells that are infectable by the said virus vector, is not functional or deleted, the vector comprising an exogenous nucleotide sequence, in its genome, encoding a transactivator protein, expressible in the said cells. With such a recombinant virus, packaging cells according to the invention can be infected. In one variation, said transactivator protein is a site-specific recombinase or comprises a functional fragment thereof, in alternative variations said transactivator protein is an RNA polymerase or comprises a transcription activator domain. Transactivator proteins that are useful in the invention include, but are not limited to, tTA, LAP, T7 RNA polymerase, Cre recombinase and FLP recombinase. In a preferred embodiment, said transactivator protein is not highly immunogenic in mammals, in particular in humans. In this context, the term "highly immunogenic" means that a low expression of said transactivator protein in cells in the body of said mammals elicits an immune response that results in a fast destruction of said cells. Said transactivator protein does preferably not require any second compound, either naturally provided by the host cell or introduced into the host cell, to promote its binding to the TRE. It is furthermore preferred that said transactivator protein is not naturally expressed in the cells used to produce the recombinant virus vector. It is preferred that the recombinant virus vector of the invention is derived from a DNA virus, where it is particularly preferred that said recombinant virus vector is derived from a virus selected from the group consisting of adenovirus, herpes simplex virus, vaccinia virus, adeno associated virus, Epstein-Barr Virus, SV-40, cytomegalovirus, and Human Papilloma Virus.

In one aspect of the invention, the recombinant virus vector lacks one or more viral genes that are naturally present in its wild-type genome, such as to, e.g., render said vector replication-deficient, to reduce immunogenicity of said vector or to create space for insertion of exogenous sequences. According to the invention, at least one of the genes that are lacking from said recombinant virus vector encodes a product, being essential for the replication of the virus in the packaging cells. In one variation, the absence of at least one viral gene other than said gene encoding a product, being essential for the replication of the virus in the packaging cell renders the recombinant virus vector replication deficient, in another variation said absence renders said vector conditionally replicating, in yet another variation the vector does not lack any gene which is essential for replication of said vector other than said gene encoding a product, being essential for the replication of the virus in the packaging cell. The advantages of using replication-deficient, conditionally replicating, or replicating vectors for various applications in, e.g., gene therapy are well understood in the art.

For the purpose of the invention, it is preferred that expression of the transactivator protein from the recombinant virus vector according to the invention is compatible with replication of said recombinant virus vector in a host cell, especially in a cell according to the invention. This means that the transactivator protein does not inhibit any process that is essential for said replication.

In one embodiment of the invention, the expression of the transactivator protein from the recombinant virus vector is driven by a constitutive promoter, in an alternative embodiment said expression is driven by a regulatable promoter The type of promoter is chosen to accomplish a useful expression profile for the transactivator protein in the context of said recombinant virus vector, such as to properly transactivate the expression of the complementing protein for replication of said recombinant virus vector. In a specific variation of the invention, said regulatable promoter is induced by in cis acting sequences on said recombinant virus vector. In another, not mutually excluding, variation of the invention, said regulatable promoter is induced as a consequence of DNA replication of said recombinant virus vector, in order to express the transactivator protein only during the late phase of the replication of said recombinant virus vector. In yet another not mutually excluding variation of the invention, said regulatable promoter is the endogenous promoter of a viral gene that is functionally deleted from said recombinant virus vector or comprises a functional fragment thereof, this will result in an expression profile of the transactivator protein that parallels the expression profile of said viral gene.

The invention furthermore provides a recombinant adenovirus vector with an expression cassette comprising an ORF encoding a transactivator protein inserted in its genome. The invention does not dictate the site of insertion in the adenovirus genome; said insertion may be at any location in the genome that does not interfere with adenovirus replication in cells complementing functionally deleted adenovirus functions in said recombinant adenovirus vector. Said recombinant adenovirus vector is e.g. an E1/E3-deleted vector with said expression cassette inserted in the E1-region or in the E3-region. These type of vectors are currently the most commonly used replication-deficient adenovirus vectors. The recombinant adenovirus vector according to the invention may furthermore be functionally deleted from one or more genes encoding one or more structural or regulatory proteins, where is preferred at least one of said deleted genes encoding a toxic protein. For example, an E1/E3-deleted vector with an expression cassette comprising an ORF encoding a transactivator protein inserted in the E1-region and furthermore functionally deleted of the gene encoding the fiber protein is provided. In another example of the invention, the ORF for said transactivator protein replaces the ORF of said functionally deleted gene, thereby placing the expression of said transactivator protein under control of the endogenous promoter of said functionally deleted gene. Consequently, the transactivator protein is expressed according to the expression profile of said functionally deleted gene. This will, in turn, result in an expression profile of the TRE-driven complementing gene that is very similar to that of said functionally deleted gene. The invention does not dictate the promoter driving the expression of the transactivator protein; any promoter that is active in the cell in which the recombinant adenovirus vector is replicating may be used in the method according to the invention. In particular aspects, however, it is preferred that said promoter is the adenovirus major late promoter (MLP), in order to transactivate the expression of the toxic protein only in the late phase of adenovirus replication. It is furthermore preferred that said expression cassette includes the adenovirus tripartite leader (TPL) sequences, because TPL-comprising transcripts are rescued from the adenovirus-induced shut-off of host cell protein synthesis. In vectors according to the invention where the MLP drives expression of the transactivator protein it is preferred that the expression cassette for said transactivator protein comprises the in cis acting sequences required to confer full transcriptional activity of the MLP during the late phase of adenovirus replication as defined by Mondesart et al. (Nucleic Acids Res. 19(1991)3221-3228), included by reference herein. A useful expression cassette for this aspect of the invention was disclosed in US5518913, included by reference herein.

In a variation of the invention, said expression cassette comprising an ORF encoding a transactivator protein that is inserted in the genome of a recombinant virus vector furthermore comprises target sites for a site-specific recombinase protein other than the transactivator protein, where said target sites are flanking an essential DNA fragment for expression of said ORF. The purpose of this variation is to provide said expression cassette with a means for inactivation. Replication of said recombinant virus vector in host cells expressing said site-specific recombinase other than the transactivator protein results in functional excision of said essential DNA fragment for expression of said ORF. In some applications of said recombinant virus vector it may be desired to obtain a final product that does not comprise a functional version of said expression cassette. As example of this variation of the invention, said expression cassette comprises the following elements linked in the following order: a first *loxP* site, a cytomegalovirus immediate early promoter, a second *loxP* site, an ORF encoding the tTA protein, and a SV-40 virus polyadenylation site. In another example of this variation of the invention, said expression cassette comprises the following elements linked in the following order: an MLP, a TPL, a first *loxP* site, a fragment comprising a SV-40 polyadenylation site, a second *loxP* site, an ORF encoding the tTA protein, and a SV-40 virus polyadenylation site. In this variation of the invention, it is preferred that replication of said recombinant virus vector in a cell where said site-specific recombinase protein is functionally present results in excision of said essential DNA fragment for expression of said ORF.

The present invention also provides a recombinant virus vector that in addition to said expression cassette comprising an ORF encoding a transactivator protein contains a second expression cassette comprising an ORF encoding a foreign protein or polypeptide inserted in its genome. The invention does not in any way limit the type of second expression cassette. In one variation, said foreign protein or polypeptide is a marker protein, such as firefly luciferase, bacterial □ -galactosidase, Green Fluorescent Protein, or a protein providing resistance to an antibiotic compound. In preferred embodiments of the invention, said foreign protein or polypeptide is a therapeutic protein or polypeptide. In several contemplated variations of the invention, said foreign protein or polypeptide is an anti-tumor agent, a toxin, an enzyme, a tumor suppressor protein, a cytokine, a growth factor, a hormone, an antibody, or comprises a functional fragment thereof. Said second expression cassette contains a stable or regulatable promoter to drive the expression of said foreign protein or polypeptide.

The invention furthermore contemplates a recombinant virus vector that carries at least one modified gene encoding a capsid or surface protein with altered structure, such as a fiber protein, where said altered structure changes the binding specificity of the recombinant virus vector or decreases the immune response against the recombinant virus vector in the body of an animal or human. Such a gene may however also be present in the packaging cell and be expressed when the said cell is infected by the virus vector.

In a further embodiment, the present invention provides a composition of a recombinant virus vector according to the invention and a cell according to the invention that carries a stable insert in its genome of a recombinant DNA construct according to the invention, where the TRE of said recombinant DNA construct comprises one or more specific binding sites for the transactivator protein encoded by said recombinant virus vector. In a preferred variation of this aspect of the invention, the expression of said transactivator protein is regulated by the endogenous promoter of the toxic protein encoded by said recombinant DNA construct. This variation is preferred, because it results in a regulation of the expression of said toxic protein that is very similar to its natural expression regulation. The invention also provides said recombinant virus vector replicating in said cell and the recombinant virus vectors produced by said cell. It is to be understood that recombinant virus vectors that replicated in cells according to the invention during at least one step of their production process, and that may subsequently be further propagated and/or purified, are also part of the present invention.

The invention also provides methods to generate the recombinant DNA constructs, recombinant virus vectors, cells, compositions, and recombinant virus vector products according to the invention. In various embodiments, the invention provides methods to generate preparations of recombinant virus vectors that functionally lack one or more viral genes, of recombinant virus vectors with a modified tropism, of recombinant virus vectors that express one or more foreign proteins, and of recombinant virus vectors that combine any of the aforementioned properties. In this respect, "functionally lacking a gene" means not carrying sequences that lead to the production of a protein that retains a naturally required function of the protein encoded by said gene.

The invention will be described in more detail by exemplifying a paradigm system for inducible complementing the expression of a toxic viral protein in stable packaging cells during replication of a human double-stranded DNA virus. The system described hereinafter provides complementation for the expression of the fiber protein of human adenovirus type 5 in human AdE1-complementing packaging cells during replication of recombinant adenovirus vectors in said cells.
As is described in example 2 below, it is extremely difficult to stable express the adenovirus fiber protein in AdE1-complementing packaging cells. Thus, the adenovirus fiber protein is a paradigm toxic viral protein according to the definition set forth herein.
The transactivation system employed to induce the fiber protein expression during vector replication is the *tet*O/tTA system. The expression of the fiber gene in the genome of adenovirus E1-complementing packaging cells is driven by a TRE consisting of *tet*O binding sites upstream of a minimal CMV promoter (examples 1 and 5). Induction of fiber protein expression in these cells by tTA protein is shown in example 6. Adenovirus vectors expressing the tTA transactivator protein according to the invention are described in examples 3 and 7. The expression of tTA is driven by the relatively weak MLP. This promoter was chosen to prevent induction of fiber expression to high levels during the early phase of adenovirus replication, which may interfere with the virus life cycle.
In the late phase of adenovirus replication the host protein synthesis is shut-off (e.g., Schneider and Schenk, Annu. Rev. Biochem. 56(1987)317-332). TPL-comprising transcripts are rescued from this suppression through increased nuclear export and enhanced translation (Logan and Schenk, Proc. Natl. Acad. Sci. USA 81(1984)3655-3659; Huang and Flint, J. Virol. 72(1998)225-235). Therefore, the tTA expression unit in the recombinant adenovirus vector and the fiber expression cassette in the cellular genome each comprise adenovirus TPL sequences to prevent expression shut-off during the late phase of adenovirus replication.
Example 4 shows expression induction of a marker protein in stable packaging cells through *tet*O/tTA transactivation during replication of an adenovirus vector expressing the tTA protein. Example 8 demonstrates fiber complementation in packaging cells during replication of fiber-deleted adenoviruses expressing tTA allowing production of said fiber-deleted adenoviruses.
It is to be understood, however, that this description is not meant to in any way limit the scope of the invention as was explained in more general terms above.

### Examples.

### Example 1. Construction of expression plasmids for stable or tetO/tTA inducible expression of adenovirus fiber protein.

Human adenovirus serotype 5 (Ad5) fiber gene expression constructs were made using PCR techniques. The complete Ad5 fiber ORF was amplified from Ad5 template DNA using upstream primer 5'-CTAATACGACTCACTATAGGCTCGAGCCACCATGAAGCGCGCAAGACCGTC-3' and downstream primer 5'-GCTCTAGAAACGATTCTTTATTCTTGGGC-3' comprising restriction sites for XhoI and XbaI (underlined), respectively. The sequence in the upstream primer preceding the ATG codon of the fiber ORF was chosen according to the Kozak consensus sequence for efficient initiation of translation in eukaryotic cells.

The amplification product was made blunt-end and cloned into the EcoRV site of expression vector pcDNA3 (Invitrogen) to create plasmid pCMV-Fib. The correct sequence of the entire insert was confirmed. In pCMV-Fib, expression of fiber protein is driven by the CMV immediate early promoter.

Plasmid pTRE-Fib was constructed by inserting the adenovirus fiber ORF containing EcoRI-XbaI fragment from plasmid pCMV-Fib into EcoRI/XbaI-digested pTRE (Clontech). This action places the expression regulation of the fiber ORF under direction of the tTA-inducible TRE.

The tripartite leader (TPL) sequence from human adenovirus serotype 2 was amplified from plasmid pMad5 (Toes et al., Proc. Natl. Acad. Sci. U.S.A. 94(1997):14660-14665) using primers 5'-CTCGAATTCACTCTCTTCCGCATCGCTG-3' and 5'-CAGGAATTCTTGCGACTGTGACTGGTTAG-3', each containing a restriction site for EcoRI (underlined). This PCR amplifies TPL sequences 1-201 (Logan and Shenk, Proc. Natl. Acad. Sci. U.S.A. 81(1984):3655-3659) adding flanking EcoRI sites. The EcoRI-digested amplification product was cloned into the EcoRI site upstream of the insert in pCMV-Fib or pTRE-Fib. Clones with the TPL insert in the correct orientation were designated pCMVtpl-Fib and pTREtpl-Fib, respectively.

### Example 2. Toxicity of adenovirus fiber protein in AdE1-complementing packaging cells.

AdE1-complementing 293 cells were co-transfected in T75 culture flasks with a 1:5 molar ratio of pTk-hyg (Clontech) and one of the constructs pCMV-Fib, pCMVtpl-Fib, or pcDNA3.1(-)mychisLacZ (Invitrogen) using Lipofectamine Plus (Life Technologies) according to the manufacturer's indications. One day after transfection, an aliquot of the cells was reseeded in a 24-well plate and after the cells had attached to the plate they were fixed in 50% vol. methanol, 50% vol. acetone. Subsequently, the cells were stained for fiber expression by immunocytochemistry, using anti fiber knob monoclonal antibody 1D6.14 (Nature Biotech. 14 (1996):1574-1578), rabbit antimouse immunoglobulin-alkaline phosphatase conjugate (Dako) and NBT/BCIP substrate (Dako). The percentage fiber expressing cells found is given in table 1. As can be seen, inclusion of the TPL sequence enhanced the expression of the fiber protein. Approximately 10% of pCMVtpl-Fib transfected 293 cells was expressing the fiber protein one day after transfection, showing that the CMVtpl-Fib expression cassette was functionally intact. The remaining cells were seeded in various dilutions into 10-cm culture dishes and one day later the culture medium was replaced with medium containing 200 microgram per milliliter hygromycin B (Boehringer Mannheim). After 10 days selection, individual hygromycin B resistant colonies were picked and expanded. Fiber expression was tested by immunocytochemistry as above, or mychisLacZ expression was analyzed using the same method with anti-myc monoclonal antibody 9E10 (Chan et al., Mol. Cell Probes 1(1987)73-82), instead of 1D6.14. As can be seen in table 1, none of the clones obtained after transfection with fiber expression constructs expressed detectable levels of fiber protein. In contrast, almost all control clones transfected with the mychisLacZ expression plasmid stable expressed the myc-tagged protein. Thus, during expansion of stable transfected cells there had been a selective loss of fiber-expressing cells. This suggests that fiber-expressing cells died due to toxicity of the fiber protein. This notion was further supported by the outcome of a series of independent transfection experiments on 293 or 911 AdE1-complementing cells where co-transfection of pTk-hyg with a fiber expression plasmid was directly compared to co-transfection of pTk-hyg with a similar luciferase expression plasmid. After selection for resistance to hygromycin B, the former transfections reproducibly yielded fewer clones (11+/-11SD versus 56+/-33SD). Altogether, these observations show that it is much more difficult to derive AdE1-complementing cells stable expressing adenovirus fiber than AdE1-complementing cells expressing a non-toxic protein such as LacZ or luciferase.

**Table 1.**

| Transient and stable expression of adenovirus fiber protein in transfected 293 packaging cells. | | |
|---|---|---|
| Construct transfected together with pTk-hyg | Percent fiber-expressing cells 1 day after transfection | #Fiber or LacZ expressing clones per #hygromycin-resistant clones analyzed |
| pCMV-Fib | 0,6 | 0/16 Fiber |
| pCMVtpl-Fib | 10,3 | 0/13 Fiber |
| pcDNA3.1(-)mychisLacZ | 0 | 0/14 Fiber |
| | (~20% LacZ pos.) | 13/14 LacZ |

### Example 3. Generation of recombinant adenovirus vectors expressing the tTA transactivator protein.

The 1.5 kb EcoRI-HindIII fragment encompassing the entire tTA ORF with downstream SV40 polyadenylation signal (pA) was isolated from pTet-Off (Clontech) and cloned into pBluescript II SK(-) (Stratagene), yielding pBStTApA. A 461 bp fragment encompassing the adenovirus MPL and TPL was amplified from plasmid pMad5 (Toes et al., Proc. Natl. Acad. Sci. U.S.A. 94(1997):14660-14665) using primers 5'-CTAAGAATGCGGCCGCGAGCGGTGTTCCGCGGTC-3' and 5'-CAGGAATTCTTGCGACTGTGACTGGTTAG-3', containing restriction sites for NotI and EcoRI (underlined), respectively. The NotI/EcoRI-digested amplification product was cloned into the NotI/EcoRI-sites immediately upstream of the tTA ORF in pBStTApA, yielding construct pBSMLPtTA. The MLP-TPL insert in pBSMLPtTA was sequenced using an M13 reverse primer annealing to the vector and was found to be correct. Subsequently, the entire 2 kb MLP-TPL-tTA-pA insert was excised with NotI and KpnI and cloned into NotI/KpnI-digested adenovirus shuttle vector pAdTrack (He et al., Proc. Natl. Acad. Sci. U.S.A.
95(1998):2509-2514).

The resulting plasmid pAdTrackMLP-tTA was digested with PacI and PmeI to obtain linear DNA encompassing the left-hand portion of the recombinant adenovirus vector genome. This DNA was co-transfected into 293 cells together with PacI-linearized pAdEasy-1 (He et al., Proc. Natl. Acad. Sci. U.S.A. 95(1998):2509-2514) comprising the right-hand part of the adenovirus vector genome in a 1:1 molar ratio, using Lipofectamine Plus (Life Technologies) transfection method according to the manufacturer's instructions. After homologous recombination between the two overlapping plasmids in 293 cells the resulting recombinant adenovirus vector AdGFPMLP-tTA was isolated and further propagated on 293 cells according to standard procedures known in the art. The AdGFPMLP-tTA vector co-expresses the tTA transactivator protein and the Enhanced Green Fluorescence Protein (EGFP) from expression cassettes inserted in the adenovirus E1-region. The adenovirus fiber gene is intact in this vector.

The negative control vector AdGFP was made by co-transfecting the parental construct pAdTrack with pAdEasy-1 into 293 cells using the same methods as were used to construct AdGFPMLP-tTA. AdGFP expresses EGFP but not tTA.

Titers of the two virus stocks in infectious units (IU) were determined by limiting dilution infection on 293 cells in triplicate and scoring EGFP expressing cells by fluorescent light microscopy over a two-week period. Absence of replication competent adenoviruses (RCA) was confirmed by infection of A549 cells and monitoring for plaque formation over a two-week period. The stocks were found to be free of RCA with a sensitivity of less than 1 RCA per 1E8 IU.

### Example 4. Demonstration of functional transactivation of a TRE-driven gene in AdE1-complementing adenovirus packaging cells by replicating AdGFPMLP-tTA vector.

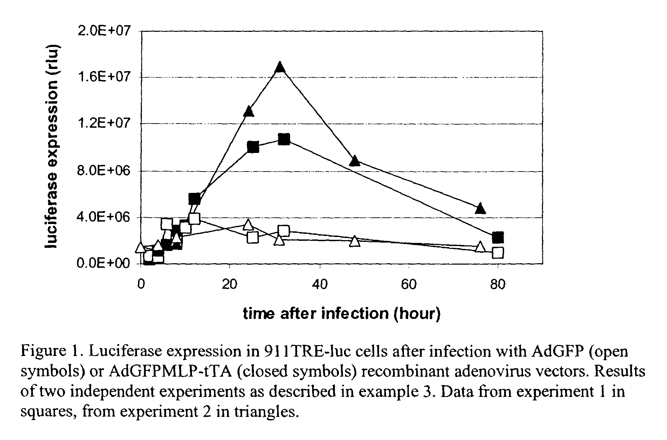

To confirm the transactivation activity of recombinant adenovirus vectors carrying an MLP-TPL-tTA-pA expression cassette in a quantitative manner, 911TRE-luc readout cells were made. To this end, 911 AdE1-complementing packaging cells were transfected with a 1:5 molar ratio of pTk-hyg and pTRE-luc (Clontech) using Lipofectamine Plus method as above. The pTRE-luc construct carries the gene encoding firefly luciferase functionally linked to the same tTA-inducible TRE as is present in the constructs pTRE-Fib and pTREtpl-Fib. Stable transfected cells were selected in medium containing 200 microgram per ml hygromycin B, and were designated 911TRE-luc cells. In these cells, the luciferase activity was measured by chemiluminescence using Luciferase Assay System (Promega) according to the manufacturer's instructions. A substantial background luciferase activity of approximately 5E8 relative light units (rlu) per mg protein was detected. A control transfection on OVCAR-3 human ovarian carcinoma cells that do not express adenovirus E1 proteins was performed to produce OVTRE-luc cells. OVTRE-luc cells exhibited an approximately 20-fold lower background luciferase expression level than did 911TRE-luc cells. These observations show that within a pool of stable transfected cells TRE-driven expression cassettes are not by definition silent in the uninduced state. Perhaps this is due to expression from the minimal CMV promoter in the TRE dependent on the insertion site within the cellular genome, where it may be influenced by neighboring enhancer sequences. The higher background in 911 cells than in OVCAR-3 cells may be caused directly or indirectly by the AdE1 expression in 911 cells.
911TRE-luc and OVTRE-luc cells were used to determine the time-course of transactivation after infection with a recombinant adenovirus vector expressing the tTA transactivator protein. In two independent experiments, 911TRE-luc and OVTRE-luc cells were infected with either AdGFPMLP-tTA or with the control vector AdGFP (see example 1) at a multiplicity of infection (m.o.i.) of 30 IU/cell. Efficient infection at the m.o.i. used was confirmed by detecting EGFP expression in all 911TRE-luc cells by fluorescent light microscopy 48 hours after infection. At various time-points after infection, the luciferase expression was measured as above. As can be seen in figure 1, the luciferase expression was induced in 911TRE-luc cells infected with AdGFPMLP-tTA reproducibly, but not in control AdGFP-infected 911TRE-luc cells. The luciferase expression started to rise approximately 12 hours after infection and reached a peak around 30 hours after infection. Thereafter, luciferase expression declined reaching background levels at the end of the experiment when cells experienced complete cytopathic effect due to virus replication. The observed induction profile reflects the adenovirus replication profile. Adenovirus DNA replication is known to start at 6-9 hours after entry of the virus into the host cell (Horwitz et al., J. Virol. 11(1973)544-551). Thus, significant transactivation was demonstrated after initiation of adenovirus DNA replication in the cells. The decline of luciferase expression after 30 hours is explained by the fact that the pTRE-luc construct lacks a TPL sequence. Thus, in 911TRE-luc cells shut-off of luciferase expression during the late phase of adenovirus replication is not prevented. OVTRE-luc cells infected with AdGFP or AdGFPMLP-tTA were analyzed over a period of 150 hours after infection. These cells remain viable after infection because E1-deleted adenovirus vectors do not replicate in these cells. No significant rise in luciferase expression was observed (not shown). This further confirms the linkage between recombinant adenovirus vector replication and transactivation activity.
In conclusion, a recombinant adenovirus vector expressing the tTA transactivator protein from a cassette with MLP and TPL is capable of functionally transactivating the expression of a TRE-driven gene inserted in the genome of host cells in which said vector is replicating. The transactivation is confined to the late phase of adenovirus replication, i.e., analogous to the natural expression profile of late adenovirus genes expressed from the endogenous MLP in the viral genome.

### Example 5. Generation of adenovirus E1-complementing packaging cells with stable pTREtpl-Fib inserts.

AdEl-complementing 293 and 911 cells were co-transfected in T75 culture flasks with a 1:5 molar ratio of pTk-hyg and pTREtpl-Fib using Lipofectamine Plus (Life Technologies) as above. As a control, pTk-hyg was co-transfected with pTRE-luc (see example 2). One day after transfection, an aliquot of the cells was reseeded in a 24-well plate and after the cells had attached to the plate they were stained for fiber expression by immunocytochemistry as above. A considerable transient fiber expression was observed in approximately 5% of pTREtpl-Fib transfected cells. This observation fits with the results described in example 4 where pTRE-luc transfected 911 cells exhibited high luciferase activity one day after transfection. The remaining cells were seeded in various dilutions into T75 culture flasks or 10-cm culture dishes and one day later the culture medium was replaced with medium containing 200 microgram per milliliter hygromycin B (Boehringer Mannheim). After 8 days selection, stable transfected pools were obtained or individual colonies were picked and expanded. Transfections with pTREtpl-Fib yielded fewer colonies than transfections with pTRE-luc (approximately 4-fold less on both 293 and 911 cells). This result may be explained by the transient expression of toxic fiber protein shortly after transfection. In total, one 293TREtpl-Fib pool, nine individual 293TREtpl-Fib clones, three 911TREtpl-Fib pools and seven individual 911TREtpl-Fib clones were established. All these cells, as well as one of the 13 pCMVtpl-Fib transfected clones from the comparative example, were subjected to PCR analysis for the presence of the fiber expression construct in their genome. As a positive control, 293 cells transfected one day before with pCMVtpl-Fib and confirmed to express fiber in approximately 1% of the cells by immunocytochemistry at the time of harvest, were included. Total genomic DNA isolated from the cells was subjected to PCR amplification using upstream primer 5'-CTCGAATTCACTCTCTTCCGCATCGCTG-3' annealing to the 5' end of the adenovirus TPL and downstream primer 5'-GCTCTAGAAACGATTCTTTATTCTTGGGC-3' annealing to the 3' end of the adenovirus fiber ORF. The underlined sequences share homology with the pCMVtpl-Fib and pTREtpl-Fib constructs. All transfected pools were shown to contain the fiber expression construct, but the intensity of the PCR signals was much lower than that of the transient transfection control. Among the transfected clones, positive and negative ones were found. Only one positive 293TREtpl-Fib clone was found, while two 293TREtpl-Fib clones carried a truncated construct and six were negative by PCR. Of the seven 911TREtpl-Fib clones four were PCR positive. Fiber expression was tested by immunocytochemistry as above. All pools and clones were found to be negative for fiber expression. Thus, as was observed for pCMVtpl-Fib transfected cells also during expansion of pTREtpl-Fib transfected cells fiber-expressing cells are lost. In conclusion, the pools and clones of stable pTREtpl-Fib transfected AdE1-complementing adenovirus packaging cells that were confirmed to carry the plasmid insert in their genome did not express the adenovirus fiber protein at detectable levels in the uninduced state, i.e., in the absence of tTA protein.

### Example 6. Induction of adenovirus fiber protein expression in 911TREtpl-Fib cells by tTA transactivator protein.

To investigate if the TREtpl-Fib insert in the genome of 911 packaging cells can functionally bind the tTA transactivator protein leading to induction of fiber protein production, one of the 911TREtpl-Fib pools growing in a 24-well culture plate was transfected with pTet-Off (Clontech) by Lipofectamine Plus method as above. Plasmid pTet-Off expresses the tTA transactivator protein directed by the CMV immediate early promoter. At 1, 2, and 3 days after transfection, the cells were analyzed for fiber expression by immunocytochemistry as above or for tTA expression using a monoclonal anti-TetR antibody (Clontech) in stead of 1D6.14. The outcome of this experiment is given in table 2. The tTA protein became detectable in transfected cells one day after transfection. Fiber expression was observed in a low number of cells (approximately 3% as compared to the number of tTA positive cells) the day thereafter. The low percentage of cells with detectable fiber expression probably reflects the relative abundance of cells within the transfected pool with a strongly inducible insert. In conclusion, this experiment shows that adenovirus fiber expression can be induced in stable 911TREtpl-Fib packaging cells when the tTA transactivator protein is expressed in these cells.

**Table 2.**

| Expression of tTA and fiber proteins in 911TREtpl-Fib cells transfected with pTet-Off as detected by immunocytochemistry. | | | | |
|---|---|---|---|---|
| Staining | Untransfected | pTet-Off transfected | | |
| | | Day 1 | Day 2 | Day 3 |
| anti-tTA | 0 | 22.3 | 26.5 | 20.5 |
| anti-Fiber | 0 | 0 | 1.2 | 0.6 |

The table gives the average number of positive cells per microscopic field inspected, a value of 0 represents no positive cell detected in a complete culture well.

### Example 7. Construction of recombinant adenovirus vectors functionally deleted of the fiber gene and expressing the tTA transactivator protein.

To derive a similar vector as AdGFPMLP-tTA, but now with a functional deletion in the fiber gene, we first made a pAdEasy-1 derivative with a large deletion in the fiber ORF. To this end, the 12 kb BamHI fragment from pAdEasy-1 was temporarily sub-cloned into a pBr322-derivative that lacks the NdeI site. In the resulting construct pBr.Ad.Bam-RITR-P the NdeI site in the fiber ORF and the AvrII site near the 3' ITR are both unique. In addition, we used the construct pBr.Ad.Bam-RITR. This construct carries the complete right-hand part of the wild-type Ad5 genome starting from the BamHI site at position 21562, cloned into the same NdeI-deficient pBr322 derivative as above. pBr.Ad.Bam-RITR was digested with NdeI and partially with Sse8387I, to delete the fragment from Ad nt 31088 to Ad nt 33288. The deleted fragment was replaced by the PCR product NN□ FS that covers the Ad5 sequence from 7 nt down-stream from the fiber ORF until the Sse8387I site, and has 5' added NdeI and NsiI sites. NN□ FS was made using primers 5'-CGACATATGTAGATGCATTAGTTTGTGTTATGTTTCAACGTG-3' (with NdeI and NsiI sites underlined) and 5'-CCTCTGGAGACGGTACAAC-3', using wild-type Ad5 DNA as template, and subsequent digestion with NdeI and Sse8387I. The resulting construct pBr.Ad.Bam-RITR□ Fib carries a unique NsiI site in place of most of the fiber ORF (approximately 1.7 kb deleted). Next, the 4.3 kb NdeI-AvrII fragment from pBr.Ad.Bam-RITR-P was replaced by the 2.6 kb NdeI-AvrII fragment from pBr.Ad.Bam-RITR□ Fib. This creates pBr.Ad.Bam-RITR□ Fib-P. BamHI-digested pBr.Ad.Bam-RITR□ Fib-P was allowed to recombine with Spellinearized pAdEasy-1 in E. coli BJ5183 cells, essentially as described by Chartier et al. (J. Virol. 70(1996):4805-4810), to create pAdEasy□ F. Finally, pAdEasy□ F was used together with pAdTrackMLP-tTA to construct pAdGFPMLP-tTA□ F, by homologous recombination in E. coli BJ5183 cells as described by He et al. (Proc. Natl. Acad. Sci. U.S.A. 95(1998):2509-2514).

### Example 8. Generation and propagation of AdGFPMLP-tTA□ F virus on TREtplFib cells.

Fiber-deleted adenovirus vectors with an expression cassette for tTA were produced by two different methods. In the first method, PacI-digested pAdGFPMLP-tTA□ F (functional recombinant adenovirus vector genome) was transfected as above into TREtpl-Fib stable transfected packaging cells. In the second method, PacI-digested pAdEasy□ F was co-transfected in a 1:1 molar ratio with PacI/PmeI-digested pAdTrackMLP-tTA into TREtpl-Fib cells as above. Three days after transfection, the transfection efficiencies were checked by fluorescence microscopy for GFP expression. In several experiments performed, approximately 20-50% transfected cells expressed GFP. Seven or 14 days after transfection, cells were scraped off the surface of the culture flasks and lysed by multiple freeze-thaw cycles in fresh culture medium containing 2% fetal bovine serum. After clearing the lysate from cell debris by centrifugation, 50% of the lysate was used to infect new TREtpl-Fib packaging cells. One week after this re-infection, viral plaques became evident in some of the cultures. This virus was further propagated on TREtpl-Fib cells according to standard procedures for adenovirus vector production known in the art.
Control transfections as above, but using pAdTrack in stead of pAdTrackMLP-tTA on TREtpl-Fib cells did not yield any viral plaques, nor did controls using pAdTrackMLP-tTA with pAdEasy□ F on conventional 293 packaging cells. Thus, the initiation of recombinant virus vector production was dependent on the functional complementation of adenovirus fiber expression through transactivation of the TREtpl-Fib insert in the host cell genome by the replicating recombinant virus vector expressing tTA.

## Claims

1. Method for the propagation of recombinant virus in packaging cells, comprising the steps of:
culturing the packaging cells free of virus
introduction of the virus in the packaging cells
propagation of the virus within the packaging cells
isolating the virus from the packaging cells,
**characterized in that**
at least one gene of the virus, the expression of which leads to a product, being essential for the replication of the virus within the packaging cells is modified to be not functional or at least partly deleted from the said virus,
the packaging cells comprise a stably replicating complementing gene, coding for a toxic complementing protein product functionally complementing for the said essential product, the expression of the said complementing gene being under the control of a transactivator responsive element (TRE),
the gene encoding the corresponding transactivator being present in the recombinant virus genome and being expressible in the packaging cells upon infection thereof by the said virus.

2. Method according to claim 1, wherein the protein, essential for the replication of the virus is toxic and encoded by the genome of the packaging cells under the control of a TRE.

3. Method according to claim 1 or 2, wherein the packaging cell in uninfected state is incapable of producing a transactivator that functionally binds the TRE.

4. Method according to any of the preceding claims, wherein the recombinant virus comprises heterologous nucleotide sequences, said heterologous sequences being chosen from the group consisting of sequences comprising the transactivator gene, sequences encoding a therapeutic product, or any combination thereof.

5. Method according to any of the preceding claims, wherein the toxic protein is a DNA virus protein or comprises a functional fragment thereof, the virus preferably being selected from the group, consisting of adenovirus, herpes simplex virus, vaccinia virus, adeno associated virus, Epstein-Barr Virus, SV40, cytomegalovirus and human Papilloma virus.

6. Method according to any of the preceding claims, wherein the toxic protein is a viral attachment protein.

7. Method according to any of the preceding claims, wherein the toxic protein is an adenovirus protein, selected from the group, consisting of hexon, fiber, penton base, 100K protein, 23K protease and core polypeptides V, VI, VII, VIII.

8. DNA construct, comprising at least one open reading frame (ORF) coding for a protein, essential for the replication of a virus, the ORF being functionally linked to a transactivator responsive element (TRE), said TRE preferably being heterologous for the said cells.

9. DNA construct according to claim 8, wherein the ORF codes for a viral attachment protein, preferably an adenovirus fiber protein.

10. DNA construct according to claim 9, wherein the nucleotide sequence of the ORF is altered such that the binding specificity of the encoded viral attachment protein for one or more different cell types is altered.

11. Cell, preferably a packaging cell, comprising the DNA construct according to any of the claims 8 - 10 as a stable insert in its genome.

12. Packaging cell according to claim 11, wherein the cell further comprises, in its genome, nucleotide sequences encoding one or more polypeptides of the E1 region of adenovirus, the packaging cell preferably being chosen from the group consisting of 293, 911 or PER cells.

13. Recombinant virus vector, of which at least one gene coding for a product being essential for replication of the virus in cells that are infectable by the said virus vector, is not functional or deleted, the vector comprising an exogenous nucleotide sequence encoding a transactivator protein, expressible in the said cells.

14. Recombinant virus vector according to claim 13, wherein the gene encoding the transactivator is operably linked to the promoter of the deleted or dysfunctional gene encoding the product, essential for virus replication.
